# EUROPEAN PATENT SPECIFICATION

(11) **EP 2 774 602 B1**
(45) Date of publication and mention of the grant of the patent: **03.06.2020**
(21) Application number: 12846271.0
(22) Date of filing: 19.09.2012
(51) Int. Cl.: A61K 8/63, A61K 8/14, A61K 8/02, A61K 9/00, A61Q 19/02, A61K 8/55, A61K 8/04, A23L 2/52

(54) **AQUEOUS DISPERSION AND METHOD FOR PRODUCING SAME**
WÄSSRIGE DISPERSION UND VERFAHREN ZU IHRER HERSTELLUNG
DISPERSION AQUEUSE ET SON PROCÉDÉ DE FABRICATION

(30) Priority: 31.10.2011 JP 2011239677; 07.06.2012 JP 2012129876
(43) Date of publication of application: 10.09.2014
(73) Proprietor: FUJIFILM Corporation, Tokyo 106-8620 (JP)
(72) Inventor: ARAKAWA, Jun, Ashigarakami-gun Kanagawa 258-8577 (JP); KANAZAWA, Katsuhiko, Ashigarakami-gun Kanagawa 258-8577 (JP); SUZUKI, Keiichi, Ashigarakami-gun Kanagawa 258-8577 (JP); NAEMURA, Masami, Ashigarakami-gun Kanagawa 258-8577 (JP); HONMA, Toshiyuki, Ashigarakami-gun Kanagawa 258-8577 (JP)
(74) Representative: Hoffmann Eitle
(86) International application number: PCT/JP2012/073928
(87) International publication number: WO 2013/065416

(56) References cited:
- EP-A1- 1 410 790
- WO-A2-2010/004099
- CN-A- 1 771 968
- DE-A1-102009 028 996
- FR-A1- 2 687 314
- JP-A- H0 446 129
- JP-A- 2002 179 516
- JP-A- 2002 338 433
- JP-A- 2004 131 502
- JP-A- 2006 036 716
- JP-A- 2006 508 968
- KR-A- 20040 027 829
- RU-C1- 2 373 924
- US-A1- 2006 034 933
- US-A1- 2006 210 619
- TECA TECA 01 September 2009, XP055149019 Retrieved from the Internet: <URL:http://www.matsumoto-trd.co. jp/product/pdf/concept/al8.pdf>

## Description

### TECHNICAL FIELD

The present invention relates to an aqueous composition and a method of producing the same.

### BACKGROUND ART

A C30 compound biosynthesized from squalene as a starting substance is called as triterpene. Triterpenes are broadly classified by a bonding mode of rings into 4 types, namely a dammarane type, a hopane type, an onocerane type and a lanostane or cycloartane type. Among them, in the dammarane type triterpene, there are tetracyclic triterpenes, such as dammarane and euphane, and pentacyclic ones, such as lupane and oleanane. The dammarane type triterpenes constitute a group having the largest number of member compounds, and among them many compounds having pharmacological action or physiological activity have been known.

Especially there are many dammarane type compounds as a main component in a vegetable extract, which exerts such functions as anti-inflammatory, humectant, whitening, anti-wrinkling, and antibacterial functions, when used in a cosmetic. Further, it has been known that a pentacyclic compound having a carboxyl group as a substituent has an especially useful function (Tanaka Nobutoshi, "Triterpene and Triterpene Type Saponin", and "Natural Product Chemistry" (Revision, 6th edition), Nankodo Co., Ltd., 1985). Such a pentacyclic triterpenes having a carboxyl group, by extracting an active ingredient from a vegetable extract ordinarily with a solvent such as ethanol, is used for a pharmaceutical, a cosmetic, a functional food, and the like.

Although a vegetable extract containing a pentacyclic triterpene as a main component is superior in functionality, the extract is generally insoluble in water and the solubility in oil is also low, which becomes a significant obstacle when the extract is added to a cosmetic or the like. Especially, when a vegetable extract containing a pentacyclic triterpene as a main component is added to a transparent cosmetic, such as a lotion, or an essence, the allowable content of the extract is restricted to a very low level since the transparency is impaired. Under such circumstances, an attainment of sufficiently high content while maintaining a transparency has been strongly desired.

Meanwhile, a particle growth with storage time tends to increase when this type of triterpene is formed to a nanoparticle dispersion in order to be transparent, there arises a problem that the turbidity increases during storage to impair the transparency conspicuously. There is also a risk that particle growth may cause coagulation or precipitation of the particle. In an extreme case, a phase separation of an active ingredient occurs. Fundamental resolution of the drawbacks has been desired.

For example, a method of resolving the drawback has been proposed, by which an oil in water emulsion having an oil droplet of 200 nm or less is formed and stabilized by additionally forming a lamellar liquid crystal film. However, by this method, it has been difficult to form an oil in water emulsion containing a pentacyclic triterpene and having a particle size of 100 nm or less, and it also has been difficult to attain a high concentration, because a large amount of fat and oil is required (see Japanese Patent No. 2650881).

Further, Japanese Patent Application Laid-Open (JP-A) No. 2008-115111 discloses a cosmetic having an emulsion form which contains a *Centella asiatica* extract and a phospholipid, and it alleges that an active ingredient such as a *Centella asiatica* extract improves skin flexibility. However, the cosmetic disclosed in JP-A-2008-115111, a micronization of dispersed particle, a transparency, an improvement in storage stability, and a correlation between a dispersion particle size and an effect such as whitening activity have not been discussed.
EP 1410790 A1 relates to a submicron-liposome containing highly concentrated triterpenoid prepared by using a non-toxic solvent without intense mechanical treatment and a method for preparing the same.
DE 10 2009 028996 A1 discloses a cosmetic formulation for the treatment of cellulite comprising caffeine, L-carnitine, at least one of a madecassoide and an asiaticoside, forskolin and an cosmetically acceptable carrier comprising lipid vesicles.
CN 1771968 A relates to an ursolic acid fat emulsion injection and its preparation. The fat emulsion consists of ursolic acid and medicinal supplementary material such as oil for injection, emulsifiers, solubilizers, isomotic agents and antioxidants.

### SUMMARY OF INVENTION

### Technical Problem

An Object of the present invention is to provide an aqueous dispersion, in which fine dispersed particles containing a pentacyclic triterpene having at least one substituent selected from the group consisting of a carboxyl group and a group derived from a carboxyl group are stably dispersed in an aqueous medium, which is superior in transparency, storage stability, and an effect derived from the pentacyclic triterpene; a method of producing the same; in addition to an aqueous composition, a cosmetic, and a functional food containing the aqueous dispersion as set forth in the claims.

### Solution to Problem

Embodiments of the present invention to solve the above-described problems are following.
<1> An aqueous composition, including: an aqueous dispersion and magnesium ascorbyl phosphate, wherein the aqueous dispersion comprises dispersed particles containing a pentacyclic triterpene derived from *Centella asiatica* extract and having at least one substituent selected from the group consisting of a carboxyl group and a group derived from a carboxyl group, and a lecithin; an amount of a phospholipid in the lecithin relative to an amount of the pentacyclic triterpene is within a range of from 2-fold to 10-fold by mass; a volume average particle diameter of the dispersed particles is from 10 nm to 70 nm, and the content of the phospholipid in the lecithin is 70 mass% or more and the content of a phosphatidylcholine in the phospholipid is in the range of from 10 to 60 mass%; wherein the pentacyclic triterpene having a group derived from a carboxyl group is selected from a glycoside type pentacyclic triterpene and a fatty acid ester type pentacyclic triterpene.
<2> The aqueous composition according to <1>, wherein a content of the aqueous dispersion is form 0.0001 mass % to 0.1 mass %.
<3> The aqueous composition according to <1> or <2>, wherein a content of the magnesium ascorbyl phosphate in the aqueous composition is from 0.1 mass % to 8 mass %.
<4> A melanin production inhibiting agent including the aqueous composition according to any one of <1> to <3>.
<5> A cosmetic including the aqueous composition according to any one of <1> to <3>.
<6> A functional food containing the aqueous composition according to any one of <1> to <3>.
<7> A method of producing the aqueous composition according to any one of <1> to <3>, including: a step of producing an aqueous dispersion, in which a suspension is obtained by mixing, with an aqueous medium, a pentacyclic triterpene derived from *Centella asiatica* extract and having at least one substituent selected from the group consisting of a carboxyl group and a group derived from a carboxyl group, and a lecithin; and conducting a dispersing treatment on the suspension at a temperature from 20°C to 80°C under a pressure not less than 100 MPa, and
   a step of mixing the aqueous dispersion and an aqueous medium including magnesium ascorbyl phosphate;
   wherein the pentacyclic triterpene having a group derived from a carboxyl group is selected from a glycoside type pentacyclic triterpene and a fatty acid ester type pentacyclic triterpene.

### Advantageous Effects of Invention

According to the present invention, an aqueous dispersion, in which fine dispersed particles containing a pentacyclic triterpene having at least one substituent selected from the group consisting of a carboxyl group and a group derived from a carboxyl group are stably dispersed, which is superior in transparency, storage stability, and an effect derived from the pentacyclic triterpene; a method of producing the same; in addition to an aqueous composition, a cosmetic, and a functional food containing the aqueous dispersion can be provided as set forth in the claims.

### Brief Description of the Drawings

Figure 1 is a graph showing the results of an examination of a melanin production inhibiting effect performed in Example 9, and Comparative Examples 4 and 5.
Figure 2 is a graph showing the results of an examination of a melanin production inhibiting effect performed in Example 15, and Comparative Examples 6 to 8

### DESCRIPTION OF EMBODIMENTS

The present invention will be described below in detail.

In the present specification, a numerical range expressed using "to" means a range including the numerical values before and after "to" as the minimum and maximum values, respectively.

In the present invention, the term "water phase" is used in contrast to a term "oil phase" irrespective of a solvent type.

In the present specification, the term "process" encompasses an independent process, as well as a process that cannot be clearly distinguished from another process but yet achieves the expected effect of the process of interest.

In a case in which the amount of a component in the composition is indicated in the present invention, when there are plural substances corresponding to the component in the composition, the indicated amount means the total amount of the plural substances present in the composition, unless specifically stated otherwise.

### [Aqueous Dispersion]

An aqueous dispersion of the present invention is an aqueous dispersion including a dispersed particle containing a pentacyclic triterpene derived from *Centella asiatica* extract and having at least one substituent selected from the group consisting of a carboxyl group and a group derived from a carboxyl group, and a lecithin; an amount of a phospholipid in the lecithin relative to an amount of the pentacyclic triterpene being within a range of from 2-fold to 10-fold by mass; and a volume average particle diameter of the dispersed particles being from 10 nm to 70 nm, and the content of the phospholipid in the lecithin is 70 mass% or more and the content of a phosphatidylcholine in the phospholipid is in the range of from 10 to 60 mass%; wherein the pentacyclic triterpene having a group derived from a carboxyl group is selected from a glycoside type pentacyclic triterpene and a fatty acid ester type pentacyclic triterpene.

An aqueous dispersion of the present invention is an oil in water (O/W) dispersion, in which dispersed particles as an oil phase are dispersed in an aqueous medium as a water phase.

An aqueous dispersion according to the present invention is an aqueous dispersion that contains, in fine dispersed particles that are stably dispersed in an aqueous medium, a pentacyclic triterpene having at least one substituent selected out of the group consisting of a carboxyl group and a group derived from a carboxyl group, and which is superior in storage stability. In other words, since, according to the present invention, a pentacyclic triterpene having at least one substituent selected out of the group consisting of a carboxyl group and a group derived from a carboxyl group is contained in dispersed particles together with a lecithin, and the phospholipid amount in the lecithin with respect to the pentacyclic triterpene amount is within a predetermined range, the pentacyclic triterpene, which conventionally has been unable to attain satisfactory water-dispersibility, can be stably dispersed in an aqueous medium, and can stably maintain such dispersibility to yield an aqueous dispersion with superior storage stability.

Further, since the dispersed particles in the present invention are fine dispersed particles with an average particle diameter from 10 nm to 70 nm, an aqueous dispersion of the present invention has high transparency.

Further, one of functions of a pentacyclic triterpene having at least one substituent selected out of the group consisting of a carboxyl group and a group derived from a carboxyl group included in the present invention is a melanin production inhibition function. Due to the pentacyclic triterpene being included in an aqueous dispersion according to the present invention, the melanin production inhibition function can be improved.

An aqueous dispersion according to the present invention can be applied to various uses such as usage in a cosmetic or a functional food, so that various functions (such as whitening, anti-inflammatory, and anti-wrinkling functions) in accordance with the functions of a pentacyclic triterpene having at least one substituent selected out of the group consisting of a carboxyl group and a group derived from a carboxyl group are able to be sufficiently and sustainably realized. An aqueous dispersion according to the present invention can be applied especially favorably to a use requiring transparency such as usage in a transparent cosmetic.

### <<Dispersed Particle>>

A dispersed particle in the present invention constitute an oil phase (dispersed phase) in an aqueous dispersion, and contains a pentacyclic triterpene having at least one substituent selected out of the group consisting of a carboxyl group and a group derived from a carboxyl group, and a lecithin.

### (Pentacyclic Triterpene)

A dispersed particle (oil phase) included in an aqueous dispersion of the present invention contains a pentacyclic triterpene derived from *Centella asiatica* extract and having at least one substituent selected from the group consisting of a carboxyl group and a group derived from a carboxyl group (hereinafter occasionally abbreviated simply as "pentacyclic triterpene") as set forth in the claims.

Since a pentacyclic triterpene in the present invention is a compound insoluble in both water and oil, it is difficult to disperse the same uniformly in water. Further, since the pentacyclic triterpene crystallizes remarkably in water, even when the same is dispersed, it has been quite difficult to maintain stably the dispersion state. Therefore it has been heretofore not possible to yield an aqueous dispersion containing a pentacyclic triterpene at a concentration sufficient for exhibiting an intended function.

In contrast, since an aqueous dispersion of the present invention includes fine dispersed particles containing a pentacyclic triterpene, it has become possible to yield a water dispersion, which contains a pentacyclic triterpene at a high concentration sufficient for exhibiting an intended function, and is also superior in storage stability.

A pentacyclic triterpene to be used in the present invention may be either of a natural product, and a synthesized product, and mainly obtainable from a vegetable extract.

Examples of a pentacyclic triterpene in the present invention include pentacyclic triterpenes having a carboxyl group or a derivative thereof as a substituent on the basic skeleton, such as an acid-type pentacyclic triterpene, a glycoside type pentacyclic triterpene, and a fatty acid ester type pentacyclic triterpene, and any of them may be applied to the present invention.

The pentacyclic triterpenes may be contained in a dispersed particle singly or in a combination of 2 or more kinds thereof in an aqueous dispersion of the present invention.

Here, an acid-type pentacyclic triterpene means a pentacyclic triterpene having a carboxyl group as a substituent on the basic skeleton.

Meanwhile, a glycoside type pentacyclic triterpene and a fatty acid ester type pentacyclic triterpene are pentacyclic triterpenes having a derivative of a carboxyl group as a substituent on the basic skeleton, and the former has on the basic skeleton a carboxyl group modified with a saccharide, and the latter has on the basic skeleton a carboxyl group modified to a fatty acid ester.

For a glycoside type pentacyclic triterpene, a monosaccharide, a disaccharide, or an oligosaccharide is preferable as a saccharide forming a glycoside. Examples thereof include monosaccharides, such as glucose, fructose, galactose, mannose, and arabinose; disaccharides, such as maltose, sucrose, and lactose; and oligosaccharides, such as malto-oligosaccharide, fructo-oligosaccharide, galacto-oligosaccharide, mannan oligosaccharide, raffinose, and stachyose.

For a fatty acid ester type, examples of an alcohol to be esterified with a carboxyl group of a pentacyclic triterpene include any and all C1 to C20 alcohols allowing combinations of saturated or unsaturated and straight chain or branched chain.

From a viewpoint of pharmacological activity, a pentacyclic triterpene in the present invention is preferably an acid-type or glycoside type pentacyclic triterpene.

Further, as an acid-type or glycoside type pentacyclic triterpene, from viewpoints of complementary manifestation of a desired function and stable dispersion of dispersed particles, it is more preferable to use at least one kind each of an acid-type pentacyclic triterpene and a glycoside type pentacyclic triterpene in a combination.

When pentacyclic triterpenes of an acid-type and a glycoside type are used in a combination, there is no particular restriction on the content ratio of an acid-type to a glycoside type, however the content ratio (molar ratio) of acid-type/glycoside type is preferably from 9/1 to 1/9, and more preferably from viewpoints of functionality and stability from 7/3 to 3/7.

Examples of an acid-type pentacyclic triterpene obtainable from a vegetable extract include, but not limited to, asiatic acid, and madecassic acid contained in a *Centella asiatica* extract; ursolic acid, oleanolic acid, and betulinic acid contained in a rosemary extract; oleanolic acid, ursolic acid, and tormentic acid contained in a perillae folium extract; and glycyrrhetinic acid contained in a glycyrrhiza extract.

Examples of a pentacyclic triterpene obtainable from a vegetable extract include, in addition to the acid-type pentacyclic triterpenes, a glycoside type pentacyclic triterpene, in which a saccharide is bonded. The glycoside type pentacyclic triterpene is a form existing richly as a storage form (called as heteroside) in a plant body, and it is known that the same easily forms an acid-type triterpene (sapogenin or aglycone) by hydrolysis or enzymatic degradation.

Examples of a glycoside type pentacyclic triterpene include, but not limited to, ursolic acid glycoside, oleanolic acid glycoside, asiaticoside, madecassoside, betulinic acid glycoside, and glycyrrhizinic acid
Specific examples of a fatty acid ester type pentacyclic triterpene include methyl ursolate, ethyl ursolate, isopropyl ursolate, butyl ursolate, benzyl ursolate, methyl oleanolate, ethyl oleanolate, propyl oleanolate, butyl oleanolate, stearyl oleanolate, oleyl oleanolate, methyl betulinate, ethyl betulinate, propyl betulinate, butyl betulinate, stearyl betulinate, methyl asiatate, ethyl asiatate, propyl asiatate, butyl asiatate, stearyl asiatate, propyl glycyrrhetinate, ethyl glycyrrhetinate, stearyl glycyrrhetinate, and isostearyl glycyrrhetinate
As a pentacyclic triterpene in the present invention, a pentacyclic triterpene originated from a *Centella asiatica* extract, which is a typical vegetable extract broadly used for a cosmetic, a pharmaceutical, and a functional food, is used.

A *Centella asiatica* extract is an extract originated from *Centella asiatica,* which is a perennial herb belonging to *Apiaceae. Centella asiatica* has been known for long around the world as a medical herb, and known by various trivial names, such as "Tsubo-kusa" in Japan, Violette Marronne (Reunion island), Gotu Kola or Indian Pennywort (India), Centella Repanda (North America), and Talapetraka (Madagascar).

A large number of products using a *Centella asiatica* extract or a component contained in the extract are on the market.

The biochemical activity of a *Centella asiatica* extract is derived from an acid-type triterpene, such as asiatic acid, and madecassic acid, and a glycoside type thereof such as asiaticoside, and madecassoside (see Japanese National-Phase Publication (JP-A) No. 2009-514907). An anti-inflammatory effect, a sedating effect, and antimicrobial effect of a *Centella asiatica* have been known for a long time, and used broadly as a wound healing agent in European herbal remedy. Further, whitening activity owing to a collagen production promotion effect or anti-tyrosinase activity of *Centella asiatica* has come to known in recent years and the same is used frequently also as a cosmetic material (see F. Bonte, et al., PLANTA MEDICA, (60) 133-135 (1994)).

In an extract of a *Labiatae* plant, such as rosemary, sage, red perilla, and green perilla, large amounts of ursolic acid, and oleanolic acid are contained. Especially, ursolic acid is present at surfaces of a leaf or a fruit and has a protective function against stimulations from outside such as ultraviolet light. Further, ursolic acid has activity for helping restoration of a collagen fiber and it has been recognized that the same has an anti-wrinkling effect or a skin elasticity improvement effect, when applied to a cosmetic.

A pentacyclic triterpene to be included in an aqueous dispersion used in the present invention is, from viewpoints of transparency and storage stability, a component contained in a *Centella asiatica* extract. Further, from a viewpoint of improvement of the effect originated from a pentacyclic triterpene, such as whitening activity, a component contained in a *Centella asiatica* extract is used, as a pentacyclic triterpene to be contained in an aqueous dispersion of the present invention.

As a pentacyclic triterpene, those represented by the following general formula (1) are preferable among the aforedescribed pentacyclic triterpenes.

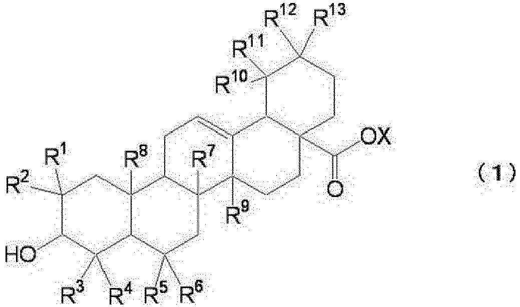

In the general formula (1), each of R¹ to R¹³ independently represents a hydrogen atom, a hydroxyl group, -CₙH₂ₙ₊₁, or -CₙH₂ₙOH, wherein n represents an integer from 1 to 5. X represents a hydrogen atom or -(Sac)ₘ, wherein Sac represents a saccharide select from the group consisting of glucose, fructose, galactose, and arabinose, and m represents a degree of polymerization from 1 to 10.

In general formula (1), each of R¹ to R¹³ is more preferably the following group:
R¹ is preferably a hydrogen atom, or a hydroxyl group.
R² is preferably a hydrogen atom.
R³ is preferably -CₙH₂ₙ₊₁ or -CₙH₂ₙOH (n=1 to 5), and more preferably -CH₃ or -CH₂OH (n=1).
R⁴ is preferably -CₙH₂ₙ₊₁ (n=1 to 5), and more preferably -CH₃ (n=1).
R⁵ is preferably a hydrogen atom or a hydroxyl group.
R⁶ is preferably a hydrogen atom.
R⁷ is preferably -CₙH₂ₙ₊₁ (n=1 to 5), and more preferably -CH₃ (n=1).
R⁸ is preferably -CₙH₂ₙ₊₁ (n=1 to 5), and more preferably -CH₃ (n=1).
R⁹ is preferably -CₙH₂ₙ₊₁ (n=1 to 5), and more preferably -CH₃ (n=1).
R¹⁰ is preferably a hydroxyl group or -CₙH₂ₙ₊₁ (n=1 to 5), and more preferably a hydroxyl group or -CH₃ (n=1).
R¹¹ is preferably a hydrogen atom.
R¹² is preferably a hydrogen atom or -CₙH₂ₙ₊₁ (n=1 to 5), and more preferably a hydrogen atom or -CH₃ (n=1).
R¹³ is preferably a hydrogen atom or -CₙH₂ₙ₊₁ (n=1 to 5), and more preferably a hydrogen atom or -CH₃ (n=1).

In general formula (1), X is preferably a hydrogen atom or -(Sac)ₘ (m=1 to 3). When X is -(Sac)ₘ (m=1 to 3), Sac is more preferably glucose.

As combinations of R1 to R13, and X in the general formula (1), combinations of the above favorable modes of R¹ to R¹³ and X are more preferable.

Examples of a favorable embodiment of a pentacyclic triterpene, among the triterpenes represented by the general formula (1), include a mixture of an acid-type triterpene represented by the following general formula (2-1) and a glycoside type triterpene represented by the following general formula (2-2).

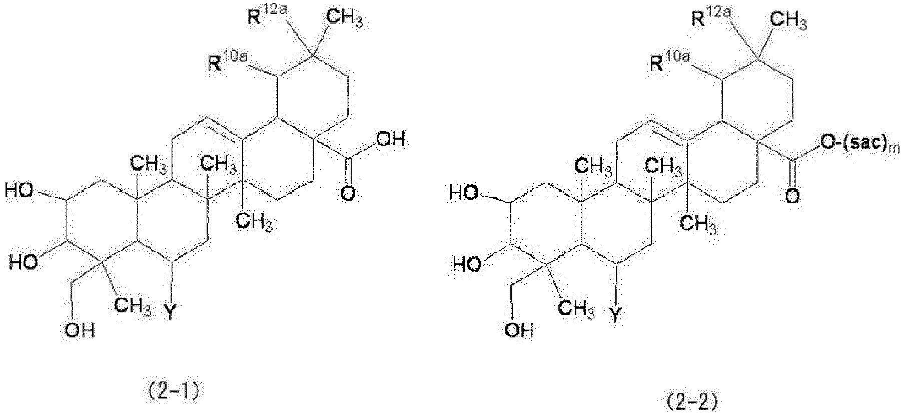

In the general formula (2-1) or (2-2), each of R^{10a} and R^{12a} independently represents a hydrogen atom or a methyl group. Y represents a hydrogen atom or a hydroxyl group. Sac and m have the same meanings as Sac and m in the general formula (1), and preferable modes are also the same.

Examples of an acid-type triterpene represented by the general formula (2-1) include asiatic acid and madecassic acid. Examples of a glycoside type triterpene represented by the general formula (2-2) include asiaticoside, and madecassoside.

The mixing ratio (molar ratio) in a mixture of an acid-type triterpene represented by the general formula (2-1) and a glycoside type triterpene represented by the general formula (2-2) is preferably from 9/1 to 1/9, and more preferably from viewpoints of functionality and stability from 7/3 to 3/7.

Exemplar compounds (compounds 1 to 8) of a triterpene represented by the general formula (1) are listed below, without being limited thereto.

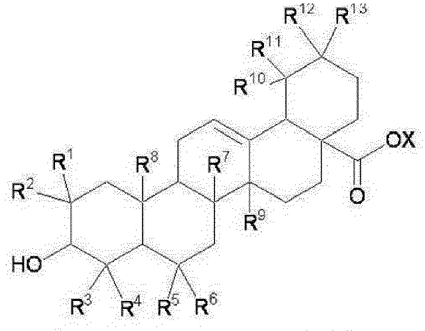

| | Compound 1 | Compound 2 | Compound 3 | Compound 4 | Compound 5 | Compound 6 | Compound 7 | Compound 8 |
|---|---|---|---|---|---|---|---|---|
| R¹ | OH | OH | OH | OH | H | H | H | H |
| R² | H | H | H | H | H | H | H | H |
| R³ | CH₂OH | CH₂OH | CH₂OH | CH₂OH | CH₃ | CH₃ | CH₃ | CH₃ |
| R⁴ | CH₃ | CH₃ | CH₃ | CH₃ | CH₃ | CH₃ | CH₃ | CH₃ |
| R⁵ | H | OH | H | OH | H | H | H | H |
| R⁶ | H | H | H | H | H | H | H | H |
| R⁷ | CH₃ | CH₃ | CH₃ | CH₃ | CH₃ | CH₃ | CH₃ | CH₃ |
| R⁸ | CH₃ | CH₃ | CH₃ | CH₃ | CH₃ | CH₃ | CH₃ | CH₃ |
| R⁹ | CH₃ | CH₃ | CH₃ | CH₃ | CH₃ | CH₃ | CH₃ | CH₃ |
| R¹⁰ | CH₃ | CH₃ | CH₃ | CH₃ | CH₃ | CH₃ | H | H |
| R¹¹ | H | H | H | H | H | H | H | H |
| R¹² | H | H | H | H | CH₃ | CH₃ | CH₃ | CH₃ |
| R¹³ | CH₃ | CH₃ | CH₃ | CH₃ | H | H | CH₃ | CH₃ |
| X | H | H | Glu₃ | Glu₃ | H | Glu | H | Glu₂ |

Among the exemplar compounds, the compounds 3, 4, 6 and 8 are embodiments, where X is "-(Sac)ₘ", "Glu" stands for glucose, and a subscript to Glu stands for a degree of polymerization "m".

Particulars of the respective compounds listed as compounds 1 to 8 are as follows.
Compound 1: Asiatic acid, available as a commercial product from Sigma-Aldrich, Inc.
Compound 2: Madecassic acid, available by extraction from a *Centella asiatica* extract.
Compound 3: Asiaticoside, available as a commercial product from Sigma-Aldrich, Inc.
Compound 4: Madecassoside, available by extraction from a *Centella asiatica* extract.
It has been known that Compounds 1, 2, 3 and 4 are contained in a *Centella asiatica* extract.

The following compounds are not derived from a *Centella asiatica* extract:
Compound 5: Ursolic acid, available as a commercial product from Tokyo Chemical Industry Co., Ltd.
Compound 6: Ursolic acid glycoside, available by synthesis according to a synthetic method pursuant to Yakugaku Zasshi, 110 (12) 958-963 (1990) using a commercial product of ursolic acid.
Compound 7: Oleanolic acid, available as a commercial product from Tokyo Chemical Industry Co., Ltd.
Compound 8: Oleanolic acid glycoside available by synthesis according to a synthetic method pursuant to Yakugaku Zasshi, 110 (12) 958-963 (1990) using a commercial product of oleanolic acid.

As a pentacyclic triterpene used in the present invention, an extract extracted from a vegetable extract can be applied directly. In this case, since plural kinds of triterpenes having different structures are ordinarily contained in a vegetable extract, plural kinds of pentacyclic triterpenes come to coexist in a dispersed particle. Such an embodiment, in which an extract extracted from a vegetable extract is used and plural kinds of pentacyclic triterpenes originated from a vegetable extract are contained in a dispersed particle, is also appropriate for practical application of an aqueous dispersion according to the present invention to a cosmetic or a function food.

Meanwhile, a pentacyclic triterpene used in the present invention may be synthesized according to a publicly known synthetic method, or may use a commercial product. Examples of a commercial product include TECA (trade name) from Bayer HealthCare AG or *Centella asiatica* extract (SEKISETSU SOU extract) from Maruzen Pharmaceuticals Co., Ltd.

The content of a pentacyclic triterpene in an aqueous dispersion in the present invention is preferably from 0.01 mass % to 10 mass % with regard to the total mass of the aqueous dispersion, more preferably from 0.05 mass % to 5 mass %, and from viewpoints of realization of high concentration of a pentacyclic triterpene in the dispersion and dispersion stability further preferably from 0.1 mass % to 3 mass %.

The content of a pentacyclic triterpene in a dispersed particle in the present invention is preferably from 9 mass % to 34 mass %, and especially preferably from 15 mass % to 30 mass %.

### (Other Oil Phase Components)

A dispersed particle (oil phase) in an aqueous dispersion of the present invention may contain various other oleosus components in addition to the pentacyclic triterpene as other oil phase components, insofar as advantageous effects of the present invention are not impaired.

Examples of other oleosus components include oleosus components, which can exhibit useful effects when applied to a cosmetic or a food. Such oleosus components can be classified from a viewpoint of a chemical structure into a fat and oil, a hydrocarbon, a wax, an ester, a fatty acid, a higher alcohol, a polymer, an oil-soluble pigmnet, an oil-soluble protein, *etc.* Other oleosus components include also a mixture of the above, various vegetable oils, and animal oils.

Specific examples of other oleosus components include a fat and oil, such as coconut oil, olive oil, corn oil, and jojoba oil, a higher fatty acid, such as stearic acid, oleic acid, palmitic acid, myristic acid, and lauric acid; a higher alcohol, such as behenyl alcohol, stearyl alcohol, and cetanol, a sterol, such as cholesterol, and phytosterol; an ester, such as ethylhexyl palmititate, isopropyl myristate, and octyldodecyl myristate; a hydrocarbon, such as squalane, hydrogenated polydecene, and hydrogenated polyisobutene.

Further, as an oleosus component having a characteristic function, a carotenoid, such as β-carotene, astaxanthin, zeaxanthin, lycopene, and lutein; a vitamin E, such as tocopherol, and tocotrienol; a ubiquinone, such as coenzyme Q10; and an ω-3 oil, such as EPA, DHA, and linolenic acid may be contained.

Further, examples of an oleosus component having a humectant function include an activity ceramide, such as ceramide I, ceramide II, ceramide III, ceramide V, and ceramide VI; a sphingo glucolipid, such as glucosyl ceramide, and galactosyl ceramide, a sphingomyelin, and a pseudo-ceramide.

When an oleosus component other than a triterpene and a lecithin is contained, the content thereof is preferably 20 mass % or less with regard to the total oleosus components in a dispersed particle containing a pentacyclic triterpene. The content of such other oleosus component is determined in accordance with an intended function.

### (Lecithin)

A dispersed particle in the present invention contains a lecithin.

A lecithin means herein a lipid mixture containing various phospholipids as main components.

Examples of phospholipids contained in the lecithin in the present invention as main components include phosphatidylcholine (PC), phosphatidylethanolamine (PE), phosphatidylinositol (PI), and phosphatidic acid (PA), without limited thereto.

The phospholipid amount in the lecithin with regard to the pentacyclic triterpene amount in accordance with the present invention is in a range of from 2-fold to 10-fold by mass, more preferably in a range of from 2-fold to 8-fold, and further preferably in a range of from 3-fold to 6-fold.

When the phospholipid amount in the lecithin by mass with regard to the pentacyclic triterpene amount is in a range of from 2-fold to 10-fold, a dispersed particle containing a pentacyclic triterpene can be dispersed stably in an aqueous medium constituting a water phase. When the lecithin amount with regard to the pentacyclic triterpene amount is less than 2-fold, sufficient dispersibility cannot be obtained, and when the same is more than 10-fold, the stability becomes insufficient.

Lecithin is a kind of glycerophospholipid, exists in all cells of plants and animals in the natural world, and is a principal constituent of a biological membrane. Therefore, lecithin can be extracted out of any plants and animals. Industrially, concerning those obtained from soybean and egg yolk as source materials, lecithin originated from soybean is called as soybean lecithin, and lecithin originated from egg yolk is called as egg yolk lecithin.

Lecithin is frequently used as an emulsifier for a food and a cosmetic owing to its safety and emulsifying power for forming an emulsion by dispersing oil in water. Further, in a pharmaceutical field lecithin is applied to a material for pharmaceutical liposome, a fat emulsion for intravenous injection, a therapeutic drug for hemorrhoid or skin disease, *etc.* utilizing an infiltration power of permeating or absorbing a substance through skin or mucosa. Soybean lecithin is broadly used for a food or a cosmetic mainly from a viewpoint of cost.

Soybean lecithin is produced by drying and purifying oily dregs by-produced in a soybean oil refining process. Pasty lecithin with a phospholipid content of ordinarily 70 mass % or less contains approx. 30 mass % of crude soybean oil. This pasty lecithin is almost exclusively used especially in a food industry field, because of its low cost. In recent years, due to needs for physiological activity of phospholipid itself or a higher quality emulsifier, a group of lecithins having various performances and functions have been developed by applying techniques, such as high degree purification, fractionation, modification, and enzymatic degradation.

Highly purified lecithin is produced by removing oil from the pasty lecithin with a solvent such as acetone followed by powderization, and contains lecithin in general at 90 mass % or more. Examples of a commercial product of the highly purified lecithin include PHOSPHOLIPON 20 (Lipoid GmbH), LECION P (Riken Vitamin Co., Ltd.), SLP WHITE (Tsuji Oil Mills Co., Ltd.), and EMULMETIK 300 (Lucas Meyer Cosmetics).

Fractionated lecithin is produced from the highly purified lecithin by increasing the content of a specific phospholipid by utilizing solubility difference in various solvents or performing an operation such as distillation. That with an enhanced content of phosphatidylcholine is in general commercially supplied. Examples of commercially available fractionated lecithin with an enhanced content of phosphatidylcholine include PHOSPHOLIPON 50 (PC content: 45 mass %), PHOSPHOLIPON 85G (PC content: 80 mass %), and PHOSPHOLIPON 90G (PC content: 94 mass %) (the foregoing are from Lipoid GmbH), EMULMETIK 900 (PC content: 50 mass %), and EMULMETIK 930 (PC content: 95 mass %) (the foregoing are from Lucas Meyer Cosmetics), SLP-PC70, and SLP-PC90 (the foregoing are from Tsuji Oil Mills Co., Ltd.).

Modified lecithin is classified broadly to hydrogenated lecithin and enzymatically degraded lecithin.

Among them, hydrogenated lecithin is prepared by converting a polyenic fatty acid in a lecithin structure to a saturated fatty acid by a hydrogenation treatment for improving the stability against oxidation or light. The hydrogenated lecithin can be used favorably for a cosmetic or a pharmaceutical. Examples of hydrogenated lecithin include EMULMETIK 320 (Lucas Meyer Cosmetics), and SLP WHITE H (Tsuji Oil Mills Co., Ltd.). Examples of a commercially available lecithin, for which the phosphatidylcholine content is enhanced and a hydrogenation treatment is carried out, include EMULMETIK 950 (Lucas Meyer Cosmetics), SLP-PC92H (Tsuji Oil Mills Co., Ltd.), and Phospholipon 90H (Lipoid GmbH).

Meanwhile, enzymatically degraded lecithin is prepared by degrading selectively an ester bond of a fatty acid at the 2nd position ordinarily bonded to glycerine, and called as lysolecithin for the sake of clear discrimination from ordinary lecithin. Lysolecithin is improved in terms of water-solubility and generally further in terms of emulsifying power, compared to original lecithin. Ordinarily the enzymatic degradation treatment is performed on original pasty lecithin, and the product is then highly purified, however, the enzymatic degradation treatment is occasionally performed also on fractionated lecithin. Examples of typical lysolecithin include SLP WHITELYSO (Tsuji Oil Mills Co., Ltd.).

As a different enzymatically treated lecithin, a lecithin, in which an ester bond between a phosphate and a base is opened up, is also produced. By the treatment a base is removed from phospholipid to form phosphatidic acid with a strong anionic character. Examples of a commercial product of this type of enzymatically treated lecithin include PA NAGASE, LYSOPHOSPHOLIPID NAGASE H (Nagase ChemteX Corporation).

Any of the above lecithins may be used in the present invention.

Among the above lecithins, a lecithin with the phospholipid content of 70 mass % or more, wherein the phosphatidylcholine content in the phospholipid is in a range of from 10 to 60 mass % is used from a viewpoint of stability over time. A lecithin with the phospholipid content of 80 mass % or more, wherein the phosphatidylcholine content in the phospholipid is in a range of from 20 to 40 mass % is more preferable.

### (Particle Size of Dispersed Particle)

The volume average particle diameter of a dispersed particle in the present invention is from 10 nm to 70 nm, preferably from 10 nm to 60 nm, and more preferably from 10 nm to 50 nm.

As the particle size of a dispersed particle in the present invention, a volume average diameter measured by a dynamic light scattering method is used from viewpoints of particle size range and measurement easiness.

Examples of a commercially-supplied measuring apparatus based on dynamic light scattering include NANOTRAC UPA (Nikkiso Co., Ltd.), dynamic light-scattering particle size analyzer LB-550 (Horiba, Ltd.), and a concentrated system particle size analyzer FPAR-1000 (Otsuka Electronics Co., Ltd.).

The volume average particle diameter of a dispersed particle in the present invention is a value measured using NANOTRAC UPA (Nikkiso Co., Ltd.), and a value measured specifically as follows is used.

Namely, for measuring the particle size, an aqueous dispersion of the present invention is diluted 10-fold with pure water, and measured at room temperature (25°C). Using the refractive index of 1.333 (pure water) for the dispersing medium and the viscosity of pure water as the viscosity for the dispersing medium, a volume average diameter is determined.

### <<Water Phase Composition>>

In an aqueous dispersion of the present invention, the dispersed particles described above as an oil phase are dispersed in an aqueous medium (dispersing medium) as a water phase.

An aqueous medium constituting a water phase in an aqueous dispersion of the present invention contains at least water, and may contain, if necessary, additionally appropriately a water-soluble substance.

### (Polyhydric Alcohol)

A water phase (dispersing medium) in an aqueous dispersion of the present invention may contain a polyhydric alcohol. A polyhydric alcohol may be used in order to improve an antiseptic property of an aqueous dispersion, or to adjust the viscosity of the same.

There is no particular restriction on a polyhydric alcohol, insofar as it is dihydric or higher alcohol. Specific examples of a polyhydric alcohol include glycerine, diglycerine, triglycerine, polyglycerine, 3-methyl-1,3-butanediol, 1,3-butylene glycol, isoprene glycol, polyethylene glycol, 1,2-pentanediol, 1,2-hexanediol, propylene glycol, dipropylene glycol, polypropylene glycol, ethylene glycol, diethylene glycol, pentaerythritol, neopentyl glycol, maltitol, reduced mizuame (starch syrup), cane sugar, lactitol, palatinit, erythritol, sorbitol, mannitol, xylitol, xylose, glucose, lactose, mannose, maltose, galactose, fructose, inositol, pentaerythritol, maltotriose, sorbitan, trehalose, starch decomposed sugar, and starch decomposed sugar reduced alcohol.

Polyhydric alcohols may be used singly or in a form of a mixture of plural kinds thereof.

A polyhydric alcohol may be used by mixing the same with water at an optional ratio, preferably at 80 mass % or less with regard to the total mass of the water phase, and more preferably at 70 mass % or less.

### (Water-Soluble Additive)

Further, a water phase (dispersing medium) in an aqueous dispersion of the present invention may contain another water-soluble additive. Examples of the water-soluble additive include a nonionic surfactant, an ionic surfactant, a water-soluble salt, a polysaccharide, a protein, a pH adjustor, an antioxidant, an antiseptic agent, a color, and a perfume, as described below.

The amount of the water-soluble additive added to water is preferably 20 mass % or less from a viewpoint of the stability of an aqueous dispersion, and more
preferably 10 mass % or less.

Examples of favorable water-soluble additives for the present invention will be described below.

### - Nonionic Surfactant -

Examples of a nonionic surfactant include a glycerin fatty acid ester, an organic acid monoglyceride, a polyglycerin fatty acid ester, a propylene glycol fatty acid ester, a polyglycerine-condensed ricinoleic acid ester, a sorbitan fatty acid ester, and a sucrose fatty acid ester, and a polyoxyethylene sorbitan fatty acid ester. Among them, a polyglycerin fatty acid ester and a sucrose fatty acid ester are preferable.

Preferable examples of a polyglycerin fatty acid ester include hexaglycerine monooleate, hexaglycerine monopalmitate, hexaglycerine monomyristate, hexaglycerine monolaurate, decaglycerine monooleate, decaglycerine monostearate, decaglycerine monopalmitate, decaglycerine monomyristate, and decaglycerine monolaurate.

Preferable examples of a sucrose fatty acid ester include sucrose dioleate, sucrose distearate, sucrose dipalmitate, sucrose dimyristate, sucrose dilaurate, sucrose monooleate, sucrose monostearate, sucrose monopalmitate, sucrose monomyristate, and sucrose monolaurate, and and among them sucrose monooleate, sucrose monostearate, sucrose monopalmitate, sucrose monomyristate, and sucrose monolaurate are more preferable.

The addition amount of a nonionic surfactant with regard to the total mass of a dispersion is preferably from 0 to 10 mass %, more preferably from 0 to 5 mass %, and further preferably 0 to 1 mass %. Further, with regard to a pentacyclic triterpene, it is preferably from 0 to 300 mass %, and more preferably from 0 to 30 mass %.

Examples of an ionic surfactant include an alkylsulfonic acid salt, an alkyl sulfate, a monoalkyl phosphoric acid salt, and a fatty acid salt.

As a water-soluble salt, sodium chloride, sodium citrate, sodium ascorbate, *etc.* are used.

Examples of a polysaccharide include maltodextrin, oligosaccharide, inulin, gum arabic, chitosan, cyclodextrin, and cluster dextrin.

Examples of a protein include various amino acids, an oligopeptide, gelatin, water-soluble collagen, and casein.

As a pH adjustor a base such as sodium hydroxide, and acid such as hydrochloric acid, and a buffer solution, such as a phosphate buffer solution, and a citrate buffer solution may be used.

Examples of an antioxidant include ascorbic acid, an ascorbic acid derivative, and monoglyceride citrate.

A small amount of a water-soluble organic solvent may, if necessary, be added in advance to a water phase of an aqueous dispersion of the present invention. In this case, the addition amount of a water-soluble organic solvent is preferably 20 mass % or less with regard to the total mass of the dispersion from a viewpoint of the stability of the dispersion over time, and more preferably 10 mass % or less.

### - Magnesium Ascorbyl Phosphate -

One appropriate water-soluble additives used in the present invention is magnesium ascorbyl phosphate. Magnesium ascorbyl phosphate is a kind of vitamin C derivative and a component having high chemical stability. Magnesium ascorbyl phosphate may be also denoted as ascorbyl phosphate magnesium, ascorbic acid 2-phosphate magnesium salt, or magnesium L-ascorbic acid phosphate.

It has been known that magnesium ascorbyl phosphate is, as soon as it is absorbed into the skin, converted to ascorbic acid by a phosphatase in the body, and exhibits a melanin production inhibiting effect.

The inventors have found a new knowledge, that coexistence of magnesium ascorbyl phosphate in an aqueous dispersion of the present invention strengthens synergistically a melanin production inhibiting effect together with various functions of a pentacyclic triterpene.

An aqueous dispersion of the present invention containing magnesium ascorbyl phosphate can be favorably applied to various uses such as a cosmetic demanding a melanin production inhibiting effect.

### (pH of Aqueous Dispersion)

The pH of an aqueous dispersion of the present invention is preferably from pH 4 to 11 from a viewpoint of the stability of dispersed particles, and more preferably from pH 5 to 8.

An aqueous dispersion of the present invention can be produced favorably by a method of producing an aqueous dispersion of the present invention described below.

### [Method of Producing Aqueous Dispersion]

An aqueous dispersion of the present invention includes obtaining a suspension by mixing, with an aqueous medium, a pentacyclic triterpene having at least one substituent selected out of the group consisting of a carboxyl group and a group derived from a carboxyl group, and a lecithin (hereinafter also referred to as "mixing process"); and conducting a dispersing treatment on the suspension at a temperature from 20°C to 80 C under a pressure not less than 100 MPa (hereinafter also referred to as "high pressure dispersion process").

Preparation of an aqueous dispersion of the present invention includes at least 2 stages of a mixing process and a high pressure dispersion process, and if necessary may include another process.

### (Mixing Process)

At the 1st stage of the mixing process a pentacyclic triterpene having at least one substituent selected from the group consisting of a carboxyl group and a group derived from a carboxyl group (pentacyclic triterpene) in the present invention, and a lecithin are mixed in aqueous medium to form a suspension. If necessary, another optional oleosus component contained in an oil phase may be admixed in addition to the pentacyclic triterpene and lecithin.

Details of a pentacyclic triterpene, a lecithin, and other oleosus components are as described above.

Further, as an aqueous medium to be used in the process, the aqueous medium constituting a water phase in an aqueous dispersion of the present invention described above may be used.

A homogeneous suspension containing a pentacyclic triterpene and a lecithin can be obtained in the mixing process.

Magnesium ascorbyl phosphate is used as an appropriate water-soluble additive in the present invention. There is no particular restriction on an addition mode, and it may be any one of a mode, in which magnesium ascorbyl phosphate is added in an aqueous medium together with a pentacyclic triterpene and a lecithin before mixing in the mixing process, a mode, in which the same is added to the liquid during mixing in the mixing process, a mode, in which the same is added to an obtained suspension after the mixing process, and a mode, in which the same is added into an obtained aqueous dispersion after a high pressure dispersion process described below.

As a mixing means to be applied in the mixing process may be any of commercially available mixing means.

Examples of a mixing means include a magnetic stirrer, a household blender, a paddle mixer, an impeller mixer, a homo-mixer, a dispersion mixer, and an ultra mixer. Among the mixing means, use of a homo-mixer, a dispersion mixer, an ultra mixer, *etc.* generating a high shear force is more preferable.

While stirring using a mixing means, a pentacyclic triterpene and a lecithin are added one by one into an aqueous medium to prepare a homogeneous suspension.

Further, it is also preferable to impart ultrasonic waves in the mixing process using an ultrasonic wave imparting means in addition to a mixing means in order to open up coagulation of dispersed particles. As a mixing means provided with an ultrasonic wave imparting means, use of an ultrasonic homogenizer is preferable.

Examples of an ultrasonic homogenizer include an ultrasonic homogenizer US-600, ditto US-1200T, ditto RUS-1200T, ditto MUS-1200T (the foregoing are from Nippon Seiki Co., Ltd.), an ultrasonic processor UIP2000, ditto UIP-4000, ditto UIP-8000, and ditto UIP-16000 (the foregoing are from Hielscher Ultrasonics GmbH). The high power ultrasonic wave irradiation apparatus are used at a frequency of 25 kHz or less, and preferably from 15 to 20 kHz. As other mixing means, means, such as a static mixer, a microchannel, and a micromixer, which have no stirring part driven from outside and require only low energy, may be also used.

Although it is possible to operate the mixing process as the 1st stage at an optional temperature from 0°C to 90°C, the temperature is preferably from 20°C to 80°C.

### (High Pressure Dispersion Process)

At the 2nd stage, the high pressure dispersion process, a suspension obtained in the mixing process is subjected to a dispersion treatment at a temperature from 20°C to 80°C and under a pressure of 100 MPa or more. By undergoing such a high pressure dispersion treatment, a micronized dispersed particle having a volume average particle diameter in a range of from 10 nm to 70 nm can be obtained.

As a dispersing means to be applied to the high pressure dispersion process, a high pressure homogenizer is used. Since a high pressure homogenizer can apply a higher shear force compared to a stirring system, micronization is possible. There are various kinds of commercially available apparatus of a high pressure homogenizer.

High pressure homogenizers can be broadly classified into a chamber type high pressure homogenizer with a fixed throttle and a homogenizing valve type high pressure homogenizer with an aperture-controllable throttle. Examples of a chamber type high pressure homogenizer (the former) include MICROFLUIDIZER (by Microfluidics), NANOMIZER (by Yoshida Kikai Co., Ltd.), and STAR BURST (by Sugino Machine Limited). Examples of a homogenizing valve type high pressure homogenizer (the latter) include a Gaulin type homogenizer (by SPX Corporation), a Rannie type homogenizer (by Rannie), a high pressure homogenizer (by GEANiro Soavi), a homogenizer (by Sanwa Engineering Co., Ltd.), a high pressure homogenizer (by Izumi Food Machinery Co., Ltd.), and an ultra-high pressure homogenizer (by IKA Works GmbH & Co. KG).

A high pressure homogenizer is provided with a very narrow chamber or throttle in a flow channel, and by sending forcibly a liquid through the narrow channel using a pump there appears a very large pressure difference between before and after the throttle. By this pressure difference as driving energy, the liquid flows through the narrow channel at a high speed equivalent to a sonic speed generating a large shear force against a wall of the flow channel, which functions as a dispersing force. A generated shear force is in proportion to an applied pressure, and the higher an applied pressure is, the higher a generated shear energy to be used for dispersion becomes. However, from a known tendency, not all the shear force is used for dispersion, and the higher the pressure becomes, the lower the energy efficiency becomes, while the portion converted to heat increases. Namely, there is a limitation on pressure increase.

In a production method of the present invention, the pressure is 100 MPa or more from a viewpoint of dispersibility (micronization), and more preferably 150 MPa or more. There is no particular restriction on the upper limit of the pressure, insofar as a high pressure dispersion treatment is exercisable. However from a viewpoint of preventing components from deterioration by temperature increase, the pressure upper side limit is 400 MPa. Further, when a commercially-supplied apparatus is used, the pressure upper side limit is 300 MPa from a viewpoint of equipment design.

The number of dispersion operations in a high pressure dispersion treatment may be once, however for higher homogeneity of a total dispersion liquid, 2 times or more is preferable, and more preferably 2 to 5 times.

The temperature before a high pressure dispersion treatment is preferably set at between 20°C and 80°C, and more preferably between 40°C to 70°C.

Right after a high pressure dispersion treatment in the high pressure dispersion process, an obtained dispersion liquid should preferably be cooled down to a predetermined temperature rapidly using a cooling means. As a cooling means any optional cooling apparatus, such as a commercially-supplied heat exchanger, may be used.

### (Another Process)

If necessary, a production method of the present invention may include a sterilization process.

Although a sterilization process may be carried out at any stage of a production method of the present invention, preferably it should be conducted as soon as possible after the high pressure dispersion process.

Examples of a sterilization method in the sterilization process includes a method by heating, such as dry heat sterilization, and steam sterilization, a method by electromagnetic waves, such as electron beam sterilization, ionizing radiation sterilization, and high-frequency sterilization, a method by chemical action, such as gas sterilization like EOG sterilization, hydrogen peroxide low-temperature plasma sterilization, and a chemical sterilization agent, and a method by separation, such as filtration sterilization. Heat sterilization (dry heat sterilization, steam sterilization) and filtration sterilization are preferable in a production method of the present invention.

### (Preparation of Powder Composition)

For the sake of enhancement of storability, an aqueous dispersion of the present invention obtained as above may be dried to form a powder composition after being prepared as a dispersion.

In the case, where an aqueous dispersion of the present invention is formed to a powder composition, a powder composition may be formed by adding a process, in which the aqueous dispersion obtained as above is dried by a drying means such as spray drying.

As a drying means a publicly known drying means may be applied, and examples thereof include natural drying, drying by heating, hot air drying, high-frequency drying, ultrasonic drying, reduced-pressure drying, vacuum drying, freeze-drying, and spray drying. The means may be used singly, or in a combination of 2 or more kinds thereof.

As a drying means, freeze-drying, by which moisture is removed from a material in a frozen condition by sublimation of ice, is preferable. Examples of a commercially-supplied freeze drier include, but not limited to, a lyophilizer VD-800F (from Taitec Corporation), FLEXI-DRY MP (from FTS Systems), DURA TOP-DURA STOP (from FTS Systems), TAKARA FREEZE-DRYER A TYPE (from Takara ATM), a desk-top lyophilizer FD-1000 (from Tokyo Rikakikai Co., Ltd.), a vacuum lyophilizer FD-550 (from Tokyo Rikakikai Co., Ltd.), and a vacuum lyophilizer (from Takara Seisakusho).

Further, from a viewpoint of the balance between production efficiency and quality, a spray drying method is preferable as a drying means. Examples of a commercially-supplied spray drier include a spray drier SD-1000 (from Tokyo Rikakikai Co., Ltd.), a spray drier L-8I (from Ohkawara Kakohki Co., Ltd.), a closed spray drier CL-12 (from Ohkawara Kakohki Co., Ltd.), a spray drier ADL310 (from Yamato Scientific Co., Ltd.), a mini-spray drier B-290 (from Buechi Labortechnik AG), PJ-MINIMAX (from Powdering Japan), and PHARMASD (from GEANiro Soavi). Further, drying and granulation may be conducted at the same time as with, for example, a fluidized bed granulation drier MP-01 (from Powrex Corp.), a fluidized bed built-in spray drier FSD (from GEANiro Soavi).

For forming an aqueous dispersion of the present invention once formed to a powder composition back to a dispersion liquid form, water corresponding to a desired concentration is added to the powder composition for reslurrying.

### [Aqueous Composition]

An aqueous composition of the present invention is one of appropriate application embodiments using an aqueous dispersion of the present invention, and obtained by mixing the aqueous dispersion of the present invention described above and an aqueous medium containing magnesium ascorbyl phosphate.

An aqueous composition of the present invention comes to a composition having, on top of various functions of a pentacyclic triterpene, a melanin production inhibiting effect synergistically intensified owing to a combination of an aqueous dispersion of the present invention and magnesium ascorbyl phosphate. Consequently, an aqueous composition of the present invention can be favorably applied to uses, such as a cosmetic, demanding a melanin production inhibiting effect, but the use of an aqueous composition of the present invention is not limited thereto.

An aqueous dispersion of the present invention is preferably used in an aqueous composition of the present invention at a content from 0.0001 mass % to 10 mass % with regard to the total mass of the aqueous composition, and more preferably at a content from 0.0005 mass % to 1 mass %. When the content of an aqueous dispersion is 0.001 mass % or more, effects of the present invention can be obtained more adequately, and when the same is 0.5 mass % or less, the applicability of an aqueous composition to an organism is improved.

Details of magnesium ascorbyl phosphate applicable of the present invention are as described above.

The content of magnesium ascorbyl phosphate with regard to the total mass of an aqueous composition of the present invention is preferably from 0.1 mass % to 8 mass %, and more preferably from 0.3 mass % to 6 mass %. When the content of magnesium ascorbyl phosphate is 0.1 mass % or more effects of the present invention can be obtained more adequately, and when the same is 8 mass % or less, the applicability of an aqueous composition to an organism is improved.

With respect to an aqueous composition of the present invention, among the aforedescribed aqueous dispersions of the present invention, an aqueous dispersion containing a pentacyclic triterpene originated from a *Centella asiatica* extract is used. By combining an aqueous dispersion of the present invention containing a pentacyclic triterpene originated from a *Centella asiatica* extract and magnesium ascorbyl phosphate, a melanin production inhibiting effect is intensified remarkably.

An aqueous composition of the present invention can be yielded by mixing an aqueous dispersion obtained through the mixing process and the high pressure dispersion process of the production method of an aqueous dispersion of the present invention and an aqueous medium containing magnesium ascorbyl phosphate. In this case the aqueous medium contains at least water and magnesium ascorbyl phosphate, and may contain various optional components to be selected according to an end use of an aqueous composition.

There is no particular restriction on a mixing means for an aqueous dispersion of the present invention and an aqueous medium containing magnesium ascorbyl phosphate, and they may be mixed in the usual manner.

### [Cosmetic]

A cosmetic of the present invention includes an aqueous composition of the present invention as an appropriate embodiment thereof.

Since an aqueous dispersion of the present invention is an aqueous dispersion, which contains a fine dispersed particle containing a pentacyclic triterpene stably dispersed in an aqueous medium, and is superior in storage stability, a cosmetic containing the same can be provided with various functions corresponding to functions of a pentacyclic triterpene (e.g. whitening, anti-inflammatory, and anti-wrinkling functions) stably for a long time period. Further, even in a case, where transparency is required, superior transparency can be exhibited and maintained for a long time period.

Further, a cosmetic of the present invention contains an aqueous composition of the present invention, because of existence of magnesium ascorbyl phosphate together with a pentacyclic triterpene, a remarkable melanin production inhibiting effect on top of various functions of a pentacyclic triterpene can be obtained.

Examples of a cosmetic include, but not limited to, a skin care cosmetic (skin lotion, serum, milky lotion, cream, cream pack mask, pack, *etc*.), a sunscreen cosmetic, a makeup cosmetic such as lip rouge and foundation, a shampooing cosmetic, a fragrance cosmetic, a liquid body cleansing preparation, a deodorant cosmetic, and an oral care cosmetic.

Although there is no particular restriction on the content of a pentacyclic triterpene in a cosmetic, the content is more preferably 0.0001 mass % or more from a viewpoint of exhibiting effectively functions of a pentacyclic triterpene.

When a cosmetic of the present invention is used in a cosmetic product, another component, which can be added to a cosmetic, may be, if necessary, added appropriately.

### [Functional Food]

A food of the present invention contains the aqueous composition. Since in the aqueous dispersion or the aqueous composition of the present invention a fine dispersed particle containing a pentacyclic triterpene is stably dispersed in an aqueous medium, imparting superior storage stability, a food containing the same can be provided with various functions corresponding to functions of a pentacyclic triterpene stably for a long time period.

Examples of a functional food not only include general foods, such as a nutrition-supplement drink, a nutrition-supplement drink, a revitalize beverage, a palatable drink, and a frozen dessert, but also include favorably nutritional supplementary foods with a shape of tablet, granule, or capsule.

In the case of a functional food, the content of a pentacyclic triterpene in the present invention may be different depending on a product type or an intended purpose, and cannot be determined *a priori.* However the content of 0.0001 mass % or more with regard to a product is sufficient. When the addition amount is 0.001 mass % or more an intended effect can be expected.

### EXAMPLES

The present invention and disclosure will be described more specifically below by way of Examples, provided that the present invention and disclosure be not limited to the following Examples. The "part" herein is based on mass unless otherwise specified.

### [Example 1] (reference)

### (Preparation of Aqueous Dispersion)

In a uniform mixture of 67 g of glycerine and 28 g of purified water, 4 g of lecithin (trade name: SLP-WHITE; by Tsuji Oil Mills Co., Ltd.), and 1 g of TECA (trade name) (by Bayer HealthCare AG, mixture containing pentacyclic triterpenes of asiatic acid, madecassic acid, and asiaticoside) were added. The mixture was then stirred using a paddle at 30°C for 30 min to obtain a suspension A.

The obtained suspension A was subjected to a high pressure dispersion treatment, in which the suspension A was passed through an ultra-high pressure disperser (trade name: STAR BURST MINI, by Sugino Machine Limited) 5 times keeping a temperature of 60°C under a pressure of 200 MPa, to obtain a transparent dispersion liquid A as an aqueous dispersion of

### Example 1.

### (Evaluation of Aqueous Dispersion)

### <Particle Size Measurement of Dispersed Particle>

The obtained transparent dispersion liquid A was diluted 10-fold with purified water, and measured for a volume average particle diameter of the dispersed particle by a dynamic light scattering particle size analyzer (trade name: NANOTRAC UPA, by Nikkiso Co., Ltd.) to find a particle diameter of 19 nm. The details of the measuring method are as described above.

### <Evaluation of Transparency>

The transparency of the transparent dispersion liquid A immediately after preparation was visually observed to find that the same was cloudlessly transparent.

### <Evaluation of Storage Stability>

### - Evaluation A -

The transparent dispersion liquid A was heated at 80°C for 30 min and the particle size of the dispersed particle was measured similarly as in the "Particle Size Measurement of Dispersed Particle" to find a particle diameter of 21 nm.

Further, the transparency of the transparent dispersion liquid A was visually observed to find that the similar level of transparency as the transparent dispersion liquid A before heating was maintained.

### - Evaluation B -

The transparent dispersion liquid A was stored at 40°C for 2 months, and then the particle size of the dispersed particle was measured similarly as "Particle Size Measurement of Dispersed Particle" to find the particle diameter of 23 nm.

Further, the transparency of the transparent dispersion liquid A was visually observed to find that the similar level of transparency as the transparent dispersion liquid A before storing was maintained.

### [Example 2] (reference)

### (Preparation of Aqueous Dispersion)

In a uniform mixture of 67 g of glycerine and 28 g of purified water, 4 g of lecithin (trade name: SLP-WHITE; by Tsuji Oil Mills Co., Ltd.), and 1 g of a rosemary extract powder (trade name: ROSEMARY T3 COMPLEX, by Sabinsa Corporation, mixture containing pentacyclic triterpenes of ursolic acid, betulinic acid, and oleanolic acid) were added. The mixture was then stirred by a homo-mixer, and then stirred using a magnetic stirrer fat 70°C for 30 min to obtain a suspension B.

The obtained suspension B was subjected to a high pressure dispersion treatment, in which the suspension B was passed through an ultra-high pressure disperser (trade name: STAR BURST MINI, by Sugino Machine Limited) 8 times keeping a temperature of 60°C under a pressure of 200 MPa, to obtain a transparent dispersion liquid B as an aqueous dispersion of Example 2.

### (Evaluation of Aqueous Dispersion)

### <Particle Size Measurement of Dispersed Particle>

The obtained transparent dispersion liquid B was diluted 10-fold with purified water, and measured for a volume average particle diameter of the dispersed particle by a dynamic light scattering particle size analyzer (trade name: NANOTRAC UPA, by Nikkiso Co., Ltd.) similarly as "Particle Size Measurement of Dispersed Particle" in Example 1 to find a particle diameter of 26 nm.

### <Evaluation of Transparency>

The transparency of the transparent dispersion liquid B immediately after preparation was visually observed to find that the same was cloudlessly transparent.

### <Evaluation of Storage Stability>

### - Evaluation A -

The transparent dispersion liquid B was heated at 80°C for a duration of 30 min and the particle size of the dispersed particle was measured similarly as in the "Particle Size Measurement of Dispersed Particle" in Example 1 to find a particle diameter of 31 nm.

Further, the transparency of the transparent dispersion liquid B was visually observed to find that the similar level of transparency as the transparent dispersion liquid B before heating was maintained.

### - Evaluation B -

The transparent dispersion liquid B was stored at 40°C for 2 months, and then the particle size of the dispersed particle was measured similarly as "Particle Size Measurement of Dispersed Particle" in Example 1 to find the particle diameter of 42 nm.

Further, the transparency of the transparent dispersion liquid B was visually observed to find that the similar level of transparency as the transparent dispersion liquid B before storing was maintained.

### [Examples 3 to 6, Comparative Examples 1 to 3]

### (Preparation of Aqueous Dispersion)

Aqueous dispersions of Examples 3 to 6 and Comparative Examples 1 to 3 were prepared similarly as in Example 1, except that the composition used for preparing the aqueous dispersion in Example 1 was changed to the compositions set forth in the following Table 1.

### (Evaluation of Aqueous Dispersion)

### <Particle Size Measurement of Dispersed Particle>

With respect to each of the obtained aqueous dispersion, the particle size of the dispersed particle was measured similarly as in the "Particle Size Measurement of Dispersed Particle" in Example 1. The results are shown in Table 1.

### <Existence or Nonexistence of Dispersed Particle with Particle Size of 1 µm or More>

Existence or nonexistence of a dispersed particle with a particle diameter of 1 µm or more was examined with respect to each of the obtained transparent dispersion liquid using a dynamic light scattering particle size analyzer (trade name: NANOTRAC UPA, by Nikkiso Co., Ltd.). The results are shown in Table 1.

### <Evaluation of Transparency>

The transparency of each transparent dispersion liquid immediately after preparation was visually observed. The results are shown in Table 1.

### <Evaluation of Storage Stability>

Each of the obtained dispersion liquid was stored at 50°C for 1 month, and then each dispersion liquid was visually observed for evaluating the storage stability. As evaluation criteria, a liquid without generation of deposit or coagulation was judged as "dispersed" and a liquid with deposit was judged as "deposited". The results are shown in Table 1.

**[Table 1]**

| | | | Example 3 (reference) | Example 4 (reference) | Example 5 (reference) | Example 6 (reference) | Comparative Example 1 | Comparative Example 2 | Comparative Example 3 |
|---|---|---|---|---|---|---|---|---|---|
| Composition | Glycerine | | 66 g | 65 g | 56 g | 61 g | 66 g | 63 g | 65 g |
| | Purified Water | | 28 g | 28 g | 28 g | 28 g | 28 g | 28 g | 28 g |
| | Lecithin | SLP-PASTE | - | - | - | - | - | 8 g | - |
| | | SLP-WHITE H | 5 g | - | - | - | - | - | - |
| | | SLP-WHITELYSO | - | 5 g | - | - | - | - | - |
| | | SLP-PC70 | - | - | - | 10 g | - | - | - |
| | | SLP-WHITE | - | - | 15g | - | 5g | - | 5 g |
| | Pentacyclic triterpene | Acid-Type | TECA | TECA | Compound 5, 7 | Compound 5, 7 | Compound 1 | TECA | Compound 5 |
| | | Glycoside Type | | | Compound 6, 8 | Compound 6, 8 | - | | Compound 6 |
| | | Total Content | 1 g | 2 g | 2 g | 2 g | 1 g | 1 g | 1 g |
| Dispersing Condition | | Pressure | 130 MPa | 180 MPa | 200 MPa | 200 MPa | 200 MPa | 200 MPa | 70 MPa |
| | | Dispersing Temperature | 60°C | 75°C | 50°C | 50°C | 60°C | 60°C | 15°C |
| | | Dispersion Pass Number | 3 | 5 | 8 | 6 | 5 | 6 | 3 |
| Particle Diameter of Dispersed Particle | | | 62 nm | 37 nm | 29 nm | 34 nm | 239 nm | 446 nm | 89 nm |
| Existence of Dispersed Particle of 1 µm or larger | | | No | No | No | No | No | No | Yes |
| Transparency (Initial, Visual) | | | Transparent | Transparent | Transparent | Transparent | Cloudy | Cloudy | Cloudy |
| Storage Stability (after 50°C, 1 month) | | | Dispersed | Dispersed | Dispersed | Dispersed | Deposited | Deposited | Deposited |

The details of pentacyclic triterpenes and lecithins set forth in Table 1 are as follows.

### <Pentacyclic Triterpenes>

- TECA (trade name): by Bayer HealthCare AG, mixture of acid-type and glycoside type pentacyclic triterpenes
- Compounds 1, 5 to 8 are pentacyclic triterpenes having the structures corresponding to the compounds 1, 5 to 8 listed above as exemplar, and the following were used in the current Examples:
   Compound 1: asiatic acid, by Sigma-Aldrich, Inc.
   Compound 5: ursolic acid, by Tokyo Chemical Industry Co., Ltd.
   Compound 6: ursolic acid glycoside, synthesized according to a synthetic method pursuant to Yakugaku Zasshi, 110 (12), 958-963 (1990) using ursolic acid (by Tokyo Chemical Industry Co., Ltd.)
   Compound 7: oleanolic acid by Tokyo Chemical Industry Co., Ltd.
   Compound 8: oleanolic acid glycoside synthesized according to a synthetic method pursuant to Yakugaku Zasshi, 110 (12), 958-963 (1990) using oleanolic acid (by Tokyo Chemical Industry Co., Ltd.)

### <Lecithins>

- SLP-paste (trade name): phospholipid content 61 mass %, by Tsuji Oil Mills Co., Ltd.
- SLP-PC70 (trade name): phospholipid content 92 mass %, phosphatidylcholine content 72 mass %, by Tsuji Oil Mills Co., Ltd.
- SLP-WHITELYSO: phospholipid content 96 mass %, phosphatidylcholine content 7 mass %, by Tsuji Oil Mills Co., Ltd.
- SLP-WHITE: phospholipid content 97 mass %, phosphatidylcholine content 28 mass %, by Tsuji Oil Mills Co., Ltd.
- SLP-WHITE H: hydrogenated SLP-WHITE, by Tsuji Oil Mills Co., Ltd.

### [Examples 7, 8] (reference)

### (Preparation of Aqueous Dispersion)

Aqueous dispersions of Examples 7 and 8 were prepared similarly as in Example 1, except that the composition used for preparing the aqueous dispersion in Example 1 was changed to the compositions set forth in the following Table 2.

### (Evaluation of Aqueous Dispersion)

### <Particle Size Measurement of Dispersed Particle>

With respect to each of the obtained aqueous dispersion, particle size measurement of a dispersed particle, evaluations of existence or nonexistence of a dispersed particle with particle diameter of 1 µm or more, and transparency, and evaluation of storage stability were carried out. The results are shown in the following Table 2.

**[Table 2]**

| | | | Example 7 (reference) | Example 8 (reference) |
|---|---|---|---|---|
| Composition | Glycerine | | 40 g | 64.5 g |
| | 1,3-Butylene glycol | | 25.5 g | - |
| | Phenoxyethanol | | 0.5 g | 0.5g |
| | Water | | 28 g | 28 g |
| | Lecithin (SLP-WHITE) | | 5 g | 5 g |
| | Pentacyclic triterpene | Acid-Type | Glycyrrhetinic acid | Betulinic acid |
| | | Glycoside Type | Glycyrrhetinic acid glycoside | Betulinic acid glycoside |
| | | (Saccharide Type /Degree of Polymerization) | (Glu/2) | (Glu/1) |
| | | Total Content | 1 g | 2 g |
| Dispersing Condition | | Pressure | 150 MPa | 170 MPa |
| | | Dispersing Temperature | 60°C | 60°C |
| | | Dispersion Pass Number | 3 | 5 |
| Particle Diameter of Dispersed Particle | | | 62 nm | 37 nm |
| Existence of Dispersed Particle of 1 µm or larger | | | No | No |
| Transparency (initial, visual) | | | Transparent | Transparent |
| Storage Stability (After 50°C, 1 month) | | | Dispersed | Dispersed |

The details of pentacyclic triterpenes and lecithin set forth in Table 2 are as follows.

### <Pentacyclic Triterpenes>

- Glycyrrhetinic acid: by Wako Pure Chemical Industries, Ltd.
- Glycyrrhetinic acid glycoside: glycyrrhizin, by Wako Pure Chemical Industries, Ltd.
- Betulinic acid: by Wako Pure Chemical Industries, Ltd.
- Betulinic acid glycoside: synthesized according to a synthetic method pursuant to Yakugaku Zasshi, 110 (12), 958-963 (1990) using betulinic acid (by Wako Pure Chemical Industries, Ltd.)

### <Lecithins>

- SLP-WHITE: by Tsuji Oil Mills Co., Ltd.
- SLP-WHITE H: by Tsuji Oil Mills Co., Ltd.

### [Example 9 (reference), and Comparative Examples 4 and 5]

A transparent dispersion having the same composition as the transparent dispersion A obtained in Example 1 was prepared, which was defined as Example 9.

A DMSO solution was prepared by dissolving TECA in dimethyl sulfoxide (DMSO) without undergoing the processes carried out in Example 1, which was used as a comparative solution of Comparative Example 4.

In the preparation of the transparent dispersion A of Example 1, a dispersion liquid with a particle diameter of 86 nm (Comparative Example 5) was prepared in the high pressure dispersion process under the conditions: pressure of 70 MP, 60°C, and dispersion number of 2 passes.

Each of the dispersion liquids or solution was adjusted to a concentration of TECA of 0.03 mass %, or DMSO of 1.0 mass % by adding water, which was then measured in terms of a melanin production amount according to the following evaluation method for examining melanin production inhibiting effect. When the melanin production inhibiting effect is excellent, excellent whitening activity can be expected.

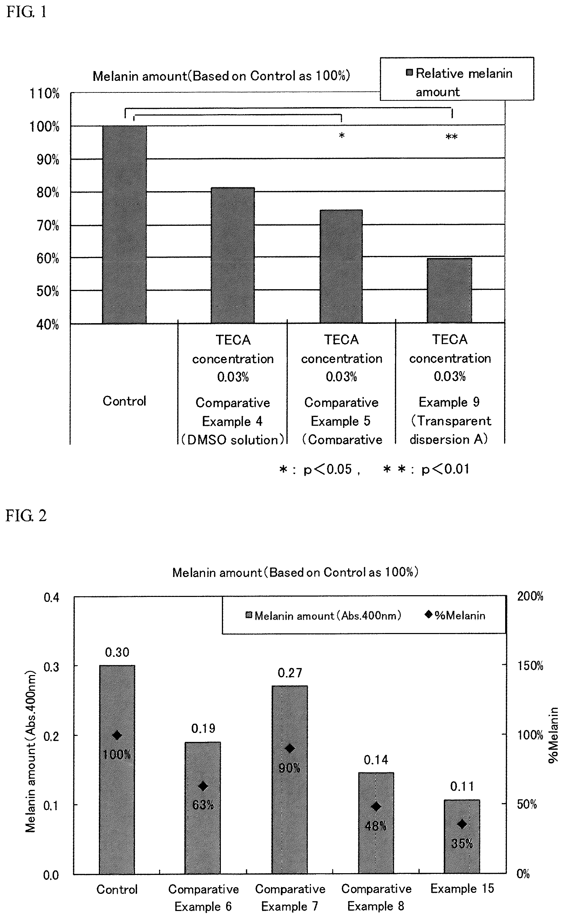

A graph showing the results is shown in Figure 1.

### - Evaluation Method -

For testing, a melanocyte containing-human three-dimensional culture epidermal model "LABCYTE MELANO-MODEL24" (by Japan Tissue Engineering Co., Ltd.) was used. This product is a human three-dimensional culture epidermal model prepared by co-culturing a human normal epidermal cell and a human normal epidermal melanocyte followed by multi-layering. The tests were conducted according to an instruction manual attached to the model.

Taking out a culture cup from an agar culture medium, culture epiderm was cultured using an improved melanin production-promoting culture medium included in a kit according to a standard protocol described in the instruction manual. Using the respective sample liquids of Example 9, Comparative Example 4, and Comparative Example 5 as test substances, 50 µL of each was seeded over a culture epiderm surface in each culture cup and cultured for 14 days. During the culture, culture medium exchange with a designated culture medium and test substance exchange in the cups were repeated every 2 to 3 days. After culturing for 14 days, measurement of cell viability (MTT method) and quantitative determination of a melanin amount (seeding of test substances was conducted n=3) were carried out according to the protocol. Meanwhile, a product of a culture conducted using only the improved melanin production promoting culture medium was defined as a control.

### - Results -

The results obtained from the above evaluations are shown in a graph in Figure 1.

With respect to the DMSO solution of Comparative Example 4, although some decreasing tendency in the melanin amount was recognizable, there was no significant difference to the control (p>0.1).

With respect to the dispersion liquid of Comparative Example 5, the melanin amount decreased significantly (p<0.05).

With respect to the dispersion liquid of Example 9, the melanin amount decreased significantly (p<0.01), and the activity was higher than the dispersion liquid of Comparative Example 5.

From the above results it was demonstrated that the dispersion liquid of Example 9 suppresses melanin production more favorably than the dispersion liquid of Comparative Example 5 and the DMSO solution of Comparative Example 4.

### [Examples 10 to 14]

Cosmetics having compositions set forth in the following Table 3 were prepared in the usual manner. The total of a composition for each Example is 100 mass %.

**[Table 3]**

| | Example | | | | |
|---|---|---|---|---|---|
| | 10 (Whitening Skin Lotion) | 11 (Anti-wrinkling Serum) (reference) | 12 (Whitening Liquid Serum) | 13 (Anti-inflammatory Skin Lotion) (reference) | 14 (Humectant Skin Lotion) (reference) |
| Aqueous Dispersion Prepared in Example 1 | 1 | - | - | - | - |
| Aqueous Dispersion Prepared in Example 2 | - | 1 | - | - | - |
| Aqueous Dispersion Prepared in Example 3 | - | - | 2 | - | - |
| Aqueous Dispersion Prepared in Example 6 | - | - | - | 1 | - |
| Aqueous Dispersion Prepared in Example 7 | - | - | - | - | 1 |
| Glycerine | 5 | 5 | 5 | 5 | 5 |
| 1,3-Butylene glycol | 6.5 | 6.5 | 6.5 | 6.5 | 6.5 |
| Polyoxyethylene sorbitan monolaurate | 1.2 | 1.2 | 1.2 | 1.2 | 1.2 |
| Ethanol | 10 | 5 | 10 | 10 | 10 |
| Tocopherol acetate | 0.01 | 0.01 | 0.01 | 0.01 | 0.01 |
| Magnesium ascorbyl phosphate | 3 | 0.2 | 0.5 | 0.5 | 0.5 |
| Carboxy vinyl polymer | 0.2 | 0.2 | - | - | 0.2 |
| Xanthan gum | - | 0.1 | 0.1 | 0.1 | - |
| Sodium citrate | 1 | 0.7 | 1 | 1 | 1 |
| Collagen | 1 | 1 | 1 | 1 | 1 |
| *N-*Acetylhydroxyproline | 1 | 1 | 1 | 1 | 1 |
| Methyl parahydroxybenzoate | 0.05 | 0.05 | 0.05 | 0.05 | 0.05 |
| Astaxanthin | 0.1 | 0.1 | 0.1 | 0.1 | 0.1 |
| Polyglyceryl-10 oleate | - | - | 0.01 | - | - |
| Sucrose fatty acid ester | - | - | 0.01 | - | - |
| Rose extract oil | - | - | 0.001 | - | - |
| Roe extract water | - | 0.001 | - | - | - |
| Polyoxyethylene hydrogenated castor oil | 0.01 | 0.01 | 0.01 | 0.01 | 0.01 |
| Lactic acid | q. s. | q. s. | q. s. | q. s. | q. s. |
| Perfume | q. s. | q. s. | q. s. | q. s. | q. s. |
| Purified water | Balance | Balance | Balance | Balance | Balance |

| | | | | | |
|---|---|---|---|---|---|
| * Either of *Euphausiacea* extract or *Haematococcus* alga extract. (mass%) | | | | | |

### [Example 15, and Comparative Examples 6 to 8]

A method of preparing test samples used in Example 15, and Comparative Examples 6 to 8 is show below.

As a test sample, an aqueous composition of Example 15 was prepared by diluting with a 10 v/v% fetal calf serum-containing MEM culture medium (GIBCO) the transparent dispersion liquid A obtained in Example 1 to a final concentration of 0.1 mass % (final TECA concentration of 0.001 mass %), and magnesium ascorbyl phosphate (L-ascorbic acid phosphate magnesium salt n-hydrate, by Wako Pure Chemical Industries, Ltd.) to a final concentration of 6 mass %.

As test samples for Comparative Examples 6 to 8, aqueous compositions were prepared by diluting with a 10 v/v% fetal calf serum-containing MEM culture medium (GIBCO) magnesium ascorbyl phosphate and/or the dispersion B (dispersion with 1 mass % of TECA dispersed in water) to a final concentration set forth in the following Table 4.

TECA was the same as in Example 3.

The 10 v/v% fetal calf serum-containing MEM culture medium (GIBCO) was used as the control.

**[Table 4]**

| Test Sample | Final Concentration | | | Melanin Amount (%) |
|---|---|---|---|---|
| | Magnesium ascorbyl phosphate | TECA | | |
| | | Addition Form | Addition Amount | |
| Control | - | - | - | 100 |
| Comparative Example 6 | 6 mass % | - | - | 63 |
| Comparative Example 7 | - | Dispersion B | 0.001 mass % | 90 |
| Comparative Example 8 | 6 mass % | Dispersion B | 0.001 mass % | 48 |
| Example 15 | 6 mass % | Dispersion A (Example 1) | 0.001 mass % | 35 |

Using the test samples (the control, the aqueous compositions of Comparative Examples 6 to 8, and Example 15), measurements of melanin production amounts, and examinations of the melanin production inhibiting effect were conducted by performing the following evaluation method (melanin production inhibition experiment). When the melanin production inhibiting effect is superior, superior whitening activity can be expected.

### <Melanin Production Suppression Experiment>

### (1) Inoculation of Cell, and Addition of Test Sample

A murine B16 melanoma cell was inoculated to a 10 v/v% fetal calf serum-containing MEM culture medium (GIBCO) on a 12-well plate at 190,000 cells/well. To the culture medium theophylline (by Wako Pure Chemical Industries, Ltd.) was added to 2 mmol/L, and then each test sample obtained above was added.

### (2) Evaluation of Melanin Amount

Cells were harvested by centrifuging each cell suspension after the cell growth test. The harvested cells were dissolved by adding thereto 300 µL of a 1 mol/L sodium hydroxide aqueous solution containing 10 v/v% dimethyl sulfoxide, and the absorbance at 400 nm of the obtained solution was measured.

The melanin amount was evaluated by a relative melanin amount (%) calculated from the absorbance of each solution with regard to the absorbance of the control as 100%

The results obtained from the above evaluations are shown in a graph in Figure 2.

As shown in Figure 2, the melanin amounts decreased in Comparative Examples 6 and 7 compared to the control, however the relative melanin amounts are beyond 50%.

Meanwhile, in Comparative Example 8, where both magnesium ascorbyl phosphate and a *Centella asiatica* extract are used, the relative melanin amount is below 50%, indicating improvement in the activity.

On the other hand, in Example 15, where the transparent dispersion A obtained in Example 1 and magnesium ascorbyl phosphate were used in a combination, the melanin amount decreased dramatically. The melanin production inhibition activity was more significant than in Comparative Example 8, and the relative melanin amount decreased to as low as 35%.

From the above results, surprisingly it has been confirmed that the aqueous composition of Example 15 containing the dispersion liquid of Example 1 and magnesium ascorbyl phosphate can synergistically intensify the melanin production inhibiting effect.

### [Example 16]

A humectant cream having the following composition was prepared in the usual manner (Total amount 100 mass %).

| [Composition] | [Content (mass %)] |
|---|---|
| Aqueous dispersion liquid prepared in Example 4 | 0.5 |
| Cetostearyl alcohol | 3.0 |
| Glycerin fatty acid ester | 2.0 |
| Polyoxyethylene (20) sorbitan monooleate | 1.0 |
| Sorbitan monostearate | 1.0 |
| Sodium *N*-stearoyl-*N*-methyl taurate | 0.5 |
| Vaseline | 5.0 |
| Dimethylpolysiloxane (100 mPa·s) | 3.0 |
| Glyceryl tri-2-ethylhexannoate | 20.0 |
| Astaxanthin | 0.05 |
| Lactic acid | 1.0 |
| Dipropylene glycol | 10.0 |
| Sodium citrate | 0.5 |
| Magnesium ascorbyl phosphate | 0.1 |
| Titanium oxide | 0.1 |
| Perfume | q. s. |
| Disodium edetate | 0.03 |
| Ethyl parahydroxybenzoate | 0.05 |
| Purified water | balance |

### [Example 17] (reference)

A soft drink having the following composition was prepared in the usual manner (Total amount 100 mass %).

| [Composition] | [Content (mass %)] |
|---|---|
| Fructose/glucose/liquid sugar | 30.0 |
| Orange fruit juice | 20.0 |
| Dispersion liquid prepared in Example 5 | 1.0 |
| Sodium citrate | 0.5 |
| Emulsifier | 0.5 |
| Perfume | q. s. |
| Purified water | balance |

### [Example 18] (reference)

A sun screen having the following composition was prepared in the usual manner (Total amount 100 mass %).

| [Composition] | [Content (mass %)] |
|---|---|
| Aqueous dispersion liquid prepared in Example 1 | 0.5 |
| Cyclopentasiloxane | 20.0 |
| Dimethicone | 10.0 |
| Titanium oxide | 5.0 |
| *t*-Butylmethoxybenzoyl-methane | 1.0 |
| Aluminum hydroxide | 1.0 |
| Isostearic acid | 0.5 |
| Sorbitan sesquioleate | 1.0 |
| Dipotassium glycyrrhetinate | 0.5 |
| Tomato fruit extract | 0.5 |
| Astaxanthin | 0.5 |
| Water-soluble collagen | 1.0 |
| Sodium citrate | 0.5 |
| Tocopherol | 0.5 |
| Aloe extract | 0.1 |
| Perfume | q. s. |
| Ethyl parahydroxybenzoate | 0.05 |
| Purified water | balance |

## Claims

1. An aqueous composition, comprising:
an aqueous dispersion and magnesium ascorbyl phosphate,
wherein
the aqueous dispersion comprises dispersed particles comprising a pentacyclic triterpene derived from Centella asiatica extract and having at least one substituent selected from the group consisting of a carboxyl group and a group derived from a carboxyl group, and a lecithin;
an amount of a phospholipid in the lecithin relative to an amount of the pentacyclic triterpene is within a range of from 2-fold to 10-fold by mass,
a volume average particle diameter of the dispersed particles is from 10 nm to 70 nm, and
the content of the phospholipid in the lecithin is 70 mass% or more and the content of a phosphatidylcholine in the phospholipid is in the range of from 10 to 60 mass%;
wherein the pentacyclic triterpene having a group derived from a carboxyl group is selected from a glycoside type pentacyclic triterpene and a fatty acid ester type pentacyclic triterpene.

2. The aqueous composition according to Claim 1, wherein a content of the aqueous dispersion is from 0.0001 mass % to 0.1 mass %.

3. The aqueous composition according to Claim 1 or 2, wherein a content of the magnesium ascorbyl phosphate in the aqueous composition is from 0.1 mass % to 8 mass %.

4. A melanin production inhibiting agent comprising the aqueous composition according to any one of Claims 1 to 3.

5. A cosmetic comprising the aqueous composition according to any one of Claims 1 to 3.

6. A functional food comprising the aqueous composition according to any one of Claims 1 to 3.

7. A method of producing the aqueous composition according to any one of Claims 1 to 3, comprising:
a step of producing an aqueous dispersion, in which a suspension is obtained by mixing, with an aqueous medium, a pentacyclic triterpene derived from *Centella asiatica* extract and having at least one substituent selected from the group consisting of a carboxyl group and a group derived from a carboxyl group, and a lecithin; and conducting a dispersing treatment on the suspension at a temperature from 20°C to 80°C under a pressure not less than 100 MPa, and
a step of mixing the aqueous dispersion and an aqueous medium comprising magnesium ascorbyl phosphate;
wherein the pentacyclic triterpene having a group derived from a carboxyl group is selected from a glycoside type pentacyclic triterpene and a fatty acid ester type pentacyclic triterpene.

## Patentansprüche

1. Wässrige Zusammensetzung, umfassend:
eine wässrige Dispersion und Magnesiumascorbylphosphat,
worin
die wässrige Dispersion dispergierte Partikel, umfassend ein pentacyclisches Triterpen, das aus einem Centella asiatica-Extrakt abgeleitet ist und zumindest einen Substituenten aufweist, ausgewählt aus der Gruppe, bestehend aus einer Carboxylgruppe und einer Gruppe, die sich aus einer Carboxylgruppe ableitet, und ein Lecithin umfasst;
wobei die Menge eines Phospholipids im Lecithin, relativ zur Menge des pentacyclischen Triterpens, massebezogen im Bereich des 2-fachen bis 10-fachen liegt,
der volumengemittelte Partikeldurchmesser der dispergierten Partikel von 10 nm bis 70 nm beträgt und
der Gehalt des Phospholipids im Lecithin 70 Masse-% oder mehr beträgt und der Gehalt eines Phosphatidylcholins im Phospholipid im Bereich von 10 bis 60 Masse-% liegt;
worin das pentacyclische Triterpen mit einer aus einer Carboxylgruppe abgeleiteten Gruppe ausgewählt ist aus einem pentacyclischen Triterpen vom Glycosidtyp und einem pentacyclischen Triterpen vom Fettsäureestertyp.

2. Wässrige Zusammensetzung gemäß Anspruch 1, worin der Gehalt der wässrigen Dispersion von 0,0001 Masse-% bis 0,1 Masse-% beträgt.

3. Wässrige Zusammensetzung gemäß Anspruch 1 oder 2, worin der Gehalt des Magnesiumascorbylphosphats in der wässrigen Zusammensetzung von 0,1 Masse-% bis 8 Masse-% beträgt.

4. Die Melaninproduktion inhibierendes Mittel, umfassend die wässrige Zusammensetzung gemäß irgendeinem der Ansprüche 1 bis 3.

5. Kosmetikum, umfassend die wässrige Zusammensetzung gemäß irgendeinem der Ansprüche 1 bis 3.

6. Funktionelles Nahrungsmittel, umfassend die wässrige Zusammensetzung gemäß irgendeinem der Ansprüche 1 bis 3.

7. Verfahren zur Herstellung der wässrigen Zusammensetzung gemäß irgendeinem der Ansprüche 1 bis 3, umfassend:
einen Schritt zur Herstellung einer wässrigen Dispersion, worin eine Suspension erhalten wird durch Vermischen eines pentacyclischen Triterpens, abgeleitet aus einem *Centella* asiatica-Extrakt und mit zumindest einem Substituenten, ausgewählt aus der Gruppe, bestehend aus einer Carboxylgruppe und einer aus einer Carboxylgruppe abgeleiteten Gruppe, mit einem wässrigen Medium und einem Lecithin; und Durchführen einer Dispergierbehandlung an der Suspension bei einer Temperatur von 20°C bis 80°C unter einem Druck von nicht kleiner als 100 MPa, und
einen Schritt zum Mischen der wässrigen Dispersion und eines wässrigen Mediums, das Magnesiumascorbylphosphat umfasst;
worin das pentacyclische Triterpen mit einer aus einer Carboxylgruppe abgeleiteten Gruppe ausgewählt ist aus einem pentacyclischen Triterpen vom Glycosidtyp und einem pentacyclischen Triterpen vom Fettsäureestertyp.

## Revendications

1. Composition aqueuse, comprenant :
une dispersion aqueuse et du phosphate d'ascorbyle de magnésium,
dans laquelle
la dispersion aqueuse comprend des particules dispersées comprenant un triterpène pentacyclique dérivé d'un extrait de *Centella asiatica* et ayant au moins un substituant sélectionné dans le groupe consistant en un groupe carboxyle et un groupe dérivé d'un groupe carboxyle, et une lécithine ;
une quantité d'un phospholipide dans la lécithine par rapport à une quantité du triterpène pentacyclique se trouve dans la plage de 2 fois à 10 fois en masse,
un diamètre de particule moyen en volume des particules dispersées est de 10 nm à 70 nm, et
la teneur en le phospholipide dans la lécithine est de 70 % en masse ou plus et la teneur en une phosphatidylcholine dans le phospholipide se trouve dans la plage de 10 à 60 % en masse ;
dans laquelle le triterpène pentacyclique ayant un groupe dérivé d'un groupe carboxyle est sélectionné parmi un triterpène pentacyclique de type glycoside et un triterpène pentacyclique de type ester d'acide gras.

2. Composition aqueuse selon la revendication 1, dans laquelle une teneur en la dispersion aqueuse est de 0,0001 % en masse à 0,1 % en masse.

3. Composition aqueuse selon la revendication 1 ou 2, dans laquelle une teneur en phosphate d'ascorbyle de magnésium dans la composition aqueuse est de 0,1 % en masse à 8 % en masse.

4. Agent d'inhibition de la production de mélanine comprenant la composition aqueuse selon l'une quelconque des revendications 1 à 3.

5. Produit cosmétique comprenant la composition aqueuse selon l'une quelconque des revendications 1 à 3.

6. Aliment fonctionnel comprenant la composition aqueuse selon l'une quelconque des revendications 1 à 3.

7. Procédé de production de la composition aqueuse selon l'une quelconque des revendications 1 à 3, comprenant :
une étape de production d'une dispersion aqueuse, dans lequel une suspension est obtenue par mélange, avec un milieu aqueux, d'un triterpène pentacyclique dérivé d'un extrait de *Centella asiatica* et ayant au moins un substituant sélectionné dans le groupe consistant en un groupe carboxyle et un groupe dérivé d'un groupe carboxyle, et une lécithine ; et la réalisation d'un traitement de dispersion sur la suspension à une température de 20 °C à 80 °C sous une pression pas inférieure à 100 MPa, et
une étape de mélange de la dispersion aqueuse et d'un milieu aqueux comprenant du phosphate d'ascorbyle de magnésium ;
dans lequel le triterpène pentacyclique ayant un groupe dérivé d'un groupe carboxyle est sélectionné parmi un triterpène pentacyclique de type glycoside et un triterpène pentacyclique de type ester d'acide gras.
